# EUROPEAN PATENT SPECIFICATION

(11) **EP 3 191 125 B1**
(45) Date of publication and mention of the grant of the patent: **20.11.2024**
(21) Application number: 15762993.2
(22) Date of filing: 11.09.2015
(51) Int. Cl.: A61K 39/00, A61P 1/12, A61P 31/14, A61K 39/215, A61K 39/39

(54) **VACCINE FOR USE IN PROTECTING OFFSPRING OF A SOW AGAINST PORCINE ENDEMIC DIARRHEA VIRUS**
IMPFSTOFF FÜR DEN SCHUTZ DER NACHKOMMENSCHAFT EINER SAU GEGEN PORCINES ENDEMISCHES DIARRHÖE-VIRUS
VACCIN DESTINÉ À PROTÉGER UNE PROGÉNITURE D'UNE TRUIE CONTRE LE VIRUS DE LA DIARRHÉE ENDÉMIQUE PORCIN

(30) Priority: 12.09.2014 EP 14184616
(43) Date of publication of application: 19.07.2017
(73) Proprietor: Intervet International B.V., 5831 AN Boxmeer (NL); Universiteit Utrecht Holding B.V., 3584 CM Utrecht (NL)
(72) Inventor: BORN VAN DEN, Erwin, NL-5831 AN Boxmeer (NL); BOSCH, Berend Jan, NL-3584 CL Utrecht (NL); ROTTIER, Peter, NL-3584 CL Utrecht (NL)
(74) Representative: Intervet International B.V.
(86) International application number: PCT/EP2015/070856
(87) International publication number: WO 2016/038197

(56) References cited:
- WO-A1-2013/152086
- ANONYMOUS: "Field study confirms protection of piglets against porcine circovirus - Company news - pig333, pig to pork community", 12 April 2011 (2011-04-12), XP093080506, Retrieved from the Internet <URL:https://www.pig333.com/company_news/field-study-confirms-protection-of-piglets-against-porcine-circovirus_4228/> [retrieved on 20230911]
- JONGSUK OH ET AL: "Immunogenicity and protective efficacy of recombinant S1 domain of the porcine epidemic diarrhea virus spike protein", ARCHIVES OF VIROLOGY, vol. 159, no. 11, 10 July 2014 (2014-07-10), pages 2977 - 2987, XP055161559, ISSN: 0304-8608, DOI: 10.1007/s00705-014-2163-7
- DAESUB SONG ET AL: "Porcine epidemic diarrhoea virus: a comprehensive review of molecular epidemiology, diagnosis, and vaccines", VIRUS GENES, KLUWER ACADEMIC PUBLISHERS, BO, vol. 44, no. 2, 22 January 2012 (2012-01-22), pages 167 - 175, XP035022160, ISSN: 1572-994X, DOI: 10.1007/S11262-012-0713-1
- SUN D B ET AL: "Spike protein region (aa 636-789) of Porcine epidemic diarrhea virus is essential for induction of neutralizing antibodies", ACTA VIROLOGICA, ACADEMIA PRAGUE, PRAGUE, CS, vol. 51, no. 3, 1 January 2007 (2007-01-01), pages 149 - 156, XP009182030, ISSN: 0001-723X
- "Vaccine Adjuvants. Preparation methods and Research Protocols", 1 January 2000, HUMANA PRESS, Totowa, New Jersey, ISBN: 978-0-89-603735-9, article ERIK B LINDBLAD: "Freund's Adjuvants", pages: 49 - 63, XP055165850
- "Der Circo-Impfstoff von Intervet", 1 June 2010 (2010-06-01), XP055165780, Retrieved from the Internet <URL:http://www.msd-tiergesundheit.de/binaries/2010-06_PorcilisPCV_One-Shot_Folder_P_tcm82-58526.pdf> [retrieved on 20150129]

## Description

### FIELD OF THE INVENTION

The present invention pertains to a vaccine for use in protecting offspring of a sow against an infection with porcine endemic diarrhea virus (PEDV) and to a method to protect offspring of a sow against an infection with PEDV.

### BACKGROUND ART

Porcine epidemic diarrhea virus (PEDV) is a member of the family Coronaviridae, genus alphacoronavirus. It is a positive-sense, enveloped, single-stranded RNA virus. Porcine epidemic diarrhea (PED) was first observed among English feeder and fattening pigs in 1971. During the 1980s and 1990s, PED was prevalent throughout Europe, in countries such as Belgium, England, Germany, France, the Netherlands, and Switzerland. These outbreaks were relatively mild and could be controlled. Currently, there are no indications that PEDV is present in Europa. Severe PEDV outbreaks with high mortality are common in Asia, where it has probably become endemic in some areas. China has seen a large increase in outbreaks since 2010 which has been attributed to the emerging of new strains, and currently PEDV is one of the main pathogens giving large economic losses in the swine industry in Asia. An Asian-like PEDV strain was for the first time introduced in the United States in April 2013 and has spread to Canada and Latin America. PEDV is most commonly transmitted via fecal-oral contact with infected swine, and may also be introduced by contaminated equipment, fomites, or personnel. Infected pigs, dirty boots, clothing, hands, equipment, or trucks can spread the disease. But there are also indications that PEDV can be transmitted through air. Sanitation and biosecurity are the best means of prevention.

In pigs, severity of disease is variable and dependent on the epidemiologic status of the herd. The primary, and often only, clinical signs are acute watery diarrhea and vomiting. In naive animals, vomiting, acute watery diarrhea, and loss of appetite in pigs of all ages can be observed; morbidity approaches 100 percent. Particularly suckling pigs are very susceptible, and they typically display watery diarrhea, dehydration, and metabolic acidosis with mortality typically between 50 and 80 percent. On the other hand, feeder and grower pigs display diarrhea, anorexia, and depression with high morbidity, but low mortality (1-3 percent). When PEDV-infected swine are introduced to a naive premises, clinical signs typically appear within 4-5 days. PEDV cannot spread to humans.

### OBJECT OF THE INVENTION

It is an object of the invention to devise a vaccine that is suitable to protect young pigs against an infection with PEDV. Vaccines intended for use in animals of all ages against an infection with PEDV are described in the art. However, actual protection is in many cases not reported, or not unambiguous reported. There is a need for a vaccine that is capable of providing in the young pig, virus neutralizing antibodies ("VN") to a level comparable to an antibody level that a typical pig obtains after wild-type infection.

### SUMMARY OF THE INVENTION

In order to meet the object of the invention, a vaccine for use in protecting offspring of a sow (the term "sow" does not exclude that the female animal is in fact a gilt) against an infection with porcine endemic diarrhea virus has been devised, the vaccine comprising non-live PEDV antigen and an oil containing adjuvant, wherein the oil is a non metabolizable oil and in that the oil is present in an amount less than 50% v/v, by administration of the vaccine to the sow at a dose of the antigen of at least 3.0E6 (i.e. 3.0×10⁶) TCID₅₀ killed whole PEDV, defined as the dose of the antigen is such that at least a VN titre is obtained with the antigen that is the same as the VN titre that can be obtained with a vaccine comprising per dose 3.0E6 TCID₅₀ killed whole PED virus.

It has been shown that with this vaccine a sow can be safely immunized and is able to reach a VN titre which is at least comparable to a typical titre obtained in an animal surviving wild type infection with PEDV. An oily adjuvant appears to be superior over a widely used alum adjuvant, and for this particular vaccine is safe (the typical minimum amount of the oil is 1%, but may be 2, 3, 4, 5, 6, 7, 8, 9, 10% or even higher). The current invention is partly based on the recognition that given the fact that the current vaccine is able to induce very high titres of VN antibodies in the vaccinated pig, off spring of a vaccinated sow will obtain the same level or even higher levels of antibodies since, as is commonly known, a sow is able to even further concentrate these antibodies in her colostrum. This, in conjunction with the fact that the sow is able to additionally provide cell mediated immunity via lactation, provides that the current vaccine is perfectly suitable to protect off spring of a vaccinated sow against an infection with PEDV through lactation (often referred to as lactogenic immunity). Preferably the sow is vaccinated when she is pregnant, but it is also possible to vaccinate the sow right before becoming pregnant. Indeed, natural level titres can protect a pig during at least four months (see: National Hog Farmer, "PEDV Immunity: Past and Present: How long does immunity last?" Sep 2, 2014 Michael Murtaugh, Cheryl Dvorak, Suzanne Stone Department of Veterinary and Biomedical Sciences, University of Minnesota, St. Paul; www.nationalhogfarmer.com) and thus, given the fact that pregnancy lasts less than 4 months, inducing high titres before becoming pregnant should be able to provide protection to the off spring via lactation, in particular when the sow is subject to repeated vaccination. For example, regular one shot vaccinations, repeated every 8, 10, 12, 14, 16, 18, 20, 22, 24 or 26 weeks (2, 2½, 3, 3½, 4, 4½, 5, 5½ or 6 months) are believed to be sufficient to induce and keep titre levels at the required level. Also, the titre may be further improved when (each of) the one shot vaccination(s) is replaced by a by a prime-boost vaccination (two vaccinations within a very short time frame of typically 2 to 4 weeks).

### DEFINITIONS

*A vaccine* is a constitution that protects against a post vaccination infection with a pathogenic micro-organism, *i.e*. a constitution that prevents or reduces the infection by the micro-organism, or against a clinical disease that results from the infection, typically by interfering with the micro-organism itself, for example via antibodies, in the vaccinated host. Vaccination thus prevents, or at least diminishes, the level of infection and/or prevents, or at least diminishes, the level of clinical disease resulting from that infection.

A safe vaccine is a vaccine that evokes a typical rectal temperature rise during the first 24 hours after administration of the vaccine below 2.0°C, preferably below 1.5°C, preferably below 1.0°C and at the same time, if it gives rise to local reactions, the reactions must be mild (swellings below 10 cm², preferably below 5 cm², more preferably below 3 cm², even more preferably below 2 cm², and most preferably below 1 cm²) and transient (disappear within 2 weeks, preferably within 1 week, more preferably within 3 days and most preferably within a day).

*Non-live porcine endemic diarrhea virus antigen* means PEDV antigen that differs from the live (replication capable) whole virus. In particular it encompasses killed virus and subunit(s) of the virus, the latter optionally being recombinantly expressed.

A *non-metabolizable* oil is an oil (a liquid substance of mineral, natural or synthetic origin that cannot be freely mixed with water) that is not metabolised by a subject animal after administration, during the lifespan of the animal (for pigs this is until the pigs is 26 - 30 weeks of age). The oil may be distributed in the animal's body, but is not degraded by a metabolic process of the animal. Examples are mineral oils such as liquid paraffin oil or heavy mineral oil, natural oils such as squalane (hydrogenated shark liver oil) and synthetic oils such as synthetic C15-C17 hydrocarbons.

*A dose of a non-live antigen corresponding to at least "X" TCID₅₀ killed whole PEDV* means that the dose of the non-live antigen (*i.e*. antigen other than replication capable whole micro organism) is such that at least a VN titre is obtained with the non-live antigen that is the same as the VN titre that can be obtained with a vaccine comprising per dose "X" TCID₅₀ killed whole PED virus.

*Killed whole virus* means an antigenic constitution that results from the killing (inactivation) of live, whole virus. This does not exclude that the viral particles are, at least partly, ruptured during the inactivation or any further downstream processing.

*A polypeptide that corresponds to (a part of) a protein* means that the polypeptide contains at least that (part of the) protein, having a sequence homology over the length of (that part of) the protein of at least 80% with the natural occurring protein, preferably at least 81, 82, 83, 84, 85, 86, 87, 88, 89, 90, 91, 92, 93, 94, 95, 96, 97, 98, 99 up to 100% sequence homology.

The *spike protein of PEDV* is a large type I transmembrane protein of about 1400 amino acids long. Like other spike proteins of corona viruses, the protein is highly glycosylated. The spike protein is organized in two domains: a N-terminal domain named S1 that is responsible for receptor binding and a C-terminal S2 domain responsible for fusion.

### EMBODIMENTS

In an embodiment of the vaccine for use according to the invention, the dose of the antigen corresponds to at least 1.5E7 TCID₅₀ killed whole PEDV. At this dose it appears to be possible to obtain titre levels that correspond to peak levels induced following wild type infection with PEDV.

In another embodiment the total amount of oil is less than 40% v/v. Lowering the amount of oil expectedly even further increases safety. Preferably the amount of oil is between 10 and 40%, more preferably between 15 and 30%.

In still another embodiment the oil is emulsified in a hydrophilic solvent. Emulsifying the oil in a hydrophilic solvent such as for example water, expectedly further increases the safety of the vaccine.

In yet another embodiment the vaccine in addition to the non-metabolizable oil comprises vitamin E acetate. This oily, biodegradable substance may further improve vaccine efficacy and stability.

### EXAMPLES

### Example 1 Virus neutralization assay

Sera were tested for the presence of neutralizing antibodies against PEDV using a virus neutralization (VN) assay on Vero cells. Briefly, serial two-fold dilutions of the test samples or reference sera were prepared in aMEM-TPB cell culture medium containing the antibiotics penicillin and streptomycin. Each dilution was mixed with an equal volume of 100 TCID₅₀ of an infectious PEDV strain that expresses green fluorescent protein (GFP) upon infection of susceptible Vero cells. After a pre-incubation period of 1 hour at 37°C, virus/serum dilution mixtures were transferred to 96-well microtitre plates containing Vero-CCL81 cells. After incubation for 48 hours at 37°C, the extent of GFP fluorescence indicative for virus replication was determined by microscopy. The antibody titre was calculated as log2 of the reciprocal of the highest serum dilution where no virus replication could be demonstrated. Obtained VN titres are presented as averages calculated from duplicate measurements.

This way, it was established that VN titres in wild-type PEDV infected pigs that survived the infection are at a level of 5.0 log2 per ml or higher, with peak levels at 8.0 to 9.0 log2.

### Example 2 The influence of the type of adjuvant

### STUDY DESIGN

Twenty piglets seronegative for PEDV were available for this trial. When the piglets were approximately five weeks old they were vaccinated via the intramuscular (IM) route, and boosted two weeks thereafter. All applications were given into the neck. The vaccines contained 1.5·10⁷ TCID₅₀ of 1 mM binary ethyleneimine (BEI) inactivated killed whole PEDV (cell culture adapted DR13) in 2 ml. Blood samples were collected right before the first vaccination, and one, two, three, four, and five weeks thereafter. Serum was collected from the blood samples and the virus neutralizing antibody titre was determined. Rectal temperatures were measured on the day of and one day after each vaccination.

The animals were divided over four groups of five animals each (see Table 1). The first group received the killed whole virus vaccine formulated in the commercially available adjuvant XSolve^{™} (available from MSD Animal Health, Boxmeer, The Netherlands), such that the final vaccine comprises 21% v/v of the non-metablizable oil Marcol^{™} 52 and 1,25% v/v of the metabolizable oil vitamin E acetate. The second group received the same antigen, formulated in the commercially available adjuvant Diluvac Forte^{™} (available from MSD Animal Health, Boxmeer, The Netherlands), such that the final vaccine comprises 7.5% v/v of the metabolizable oil vitamin E acetate. The third group received the same antigen formulated in the alum hydrogel containing vaccine ProSystem^{™} CE (available from Merck Animal Health, USA). The fourth group received the same antigen formulated in water without adjuvant.

**Table 1. Overview of animal groups and their treatment**

| Group | # piglets | PEDV antigen | Dose | Adjuvant |
|---|---|---|---|---|
| 1 | 5 | Killed whole virus | 1.5E7 TCID₅₀ | XSolve |
| 2 | 5 | Killed whole virus | 1.5E7 TCID₅₀ | Diluvac Forte |
| 3 | 5 | Killed whole virus | 1.5E7 TCID₅₀ | ProSystem CE |
| 4 | 5 | Killed whole virus | 1.5E7 TCID₅₀ | - |

### RESULTS

### Body temperature

An overview of the mean body temperatures per group are shown in Table 2. It can be concluded that none of the vaccines caused a serious temperature elevation of more the 1 °C, and that XSolve induced the largest temperature increase of the three adjuvants tested. The highest individual temperature increase observed in this group was 1.7°C.

**Table 2. Mean rectal temperatures taken after the first or second vaccination.**

| | | **Temperature after 1^{st} vaccination** | | | **Temperature after 2^{nd} vaccination** | | |
|---|---|---|---|---|---|---|---|
| **Group** | **Vaccine** | **t=0h** | **t=4h** | **t=24h** | **t=0h** | **t=4h** | **t=24h** |
| 1 | XSolve | 39.60 | 40.02 | 39.94 | 39.94 | 40.72 | 40.20 |
| 2 | Diluvac Forte | 39.90 | 40.14 | 39.68 | 40.03 | 40.60 | 39.94 |
| 3 | ProSystem CE | 39.54 | 40.16 | 39.76 | 39.89 | 40.18 | 39.84 |
| 4 | No adjuvant | 39.90 | 39.84 | 39.52 | 39.76 | 40.31 | 39.88 |

### Virus neutralization assay

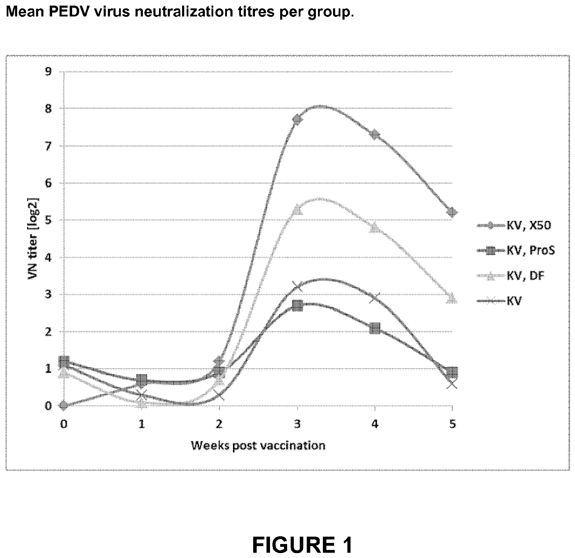

The calculated mean VN titres of each group are graphically presented in Figure 1. It can be concluded that killed whole virus antigen is able to induce good VN titres. The contribution by the adjuvants is such that the adjuvant based on the non metabilizable oil gives the highest titres, followed by the adjuvant based on the metabolizable oil, thereafter followed by the adjuvant based on alum hydroxide gel.

### Example 3 The influence of the type of non-live antigen

### STUDY DESIGN

Sixty piglets seronegative for PEDV were available for this trial. When the piglets were approximately five weeks old they were immunized with 2 ml vaccine via the intramuscular (IM) route, and boosted three weeks thereafter. All applications were given into the neck. An overview of the experimental groups is given in Table 3. Blood samples were collected before the first vaccination, and two, three, four, five, and six weeks thereafter. Serum was collected from the blood samples and the virus neutralizing antibody titre was determined. Rectal temperatures were measured on the day of vaccination and 4 and 24 hours thereafter. After each vaccination all piglets were palpated at two, four, six, eight, ten, 12, and 14 days post vaccination on the site of administration.

**Table 3. Overview of animal groups and their treatment**

| Group | # piglets | Non live PEDV antigen | | |
|---|---|---|---|---|
| | | Name | Type | Dose (in 2 ml) |
| 1a | 5 | US S1 | full-length S1 of US strain | 10 µg |
| 1b | 5 | US S1-C | C-domain of S1 of US strain | 10 µg |
| 2a | 5 | US S1-pFc | full-length S1 of US strain + Fc domain of porcine IgG | 10 µg |
| 2b | 5 | US S1-Fc | full-length S1 of US strain + Fc domain of human IgG | 10 µg |
| 3a | 5 | US S1-C-Fc | C-domain of Spike of US strain + Fc domain of human IgG | 10 µg |
| 3b | 5 | US S1-N-Fc | N-domain of Spike of US strain + Fc domain of human IgG | 10 µg |
| 4 | 10 | DR13 KV | 1 mM BEI inactivated caDR13-ESMN-ΔORF3 | 6.0·10⁵ TCID₅₀ |
| 5 | 10 | DR13 KV | 1 mM BEI inactivated caDR13-ESMN-ΔORF3 | 3.0·10⁶TCID₅₀ |
| 6 | 10 | DR13 KV | 1 mM BEI inactivated caDR13-ESMN-ΔORF3 | 1.5·10⁷ TCID₅₀ |

### MATERIALS AND METHODS

### Subunit vaccines

The antigens present in the subunit vaccines used in this study were based on the S1 domain of the PEDV spike (S) protein. The S1 amino acid sequence was derived from a US isolate which is identical to the index strain USA/Colorado/2013. The full-length S1 domain as well as the N-terminal one-third or C-terminal two-third of the S1 domain were used. To facilitate protein purification and to improve immunogenicity, some S1 proteins were C-terminally tagged with an Fc fragment derived from either human or porcine immunoglobulins. All proteins were expressed in HEK293T cells, purified, and the protein content was determined. Briefly, the HEK293T cells were transfected with expression plasmids encoding the required polypeptide of PEDV (optionally with a mouse Fc tag). One day after transfection, cell culture fluid was replaced by fresh medium. Six days after transfection, the cell culture medium was collected and cleared by centrifugation. The polypeptide was subsequently purified by binding to protein-A sepharose beads. After allowing the polypeptide to bind to the beads, it was washed three times using PBS, and the polypeptide was eluted in 0.5M HAc pH 3. The pH was neutralized by using 3M Tris pH 8.8 (final pH 7.5). Finally, the polypeptide concentration was determined at OD₂₈₀ using a nanodrop. The polypeptide was stored at -80°C.

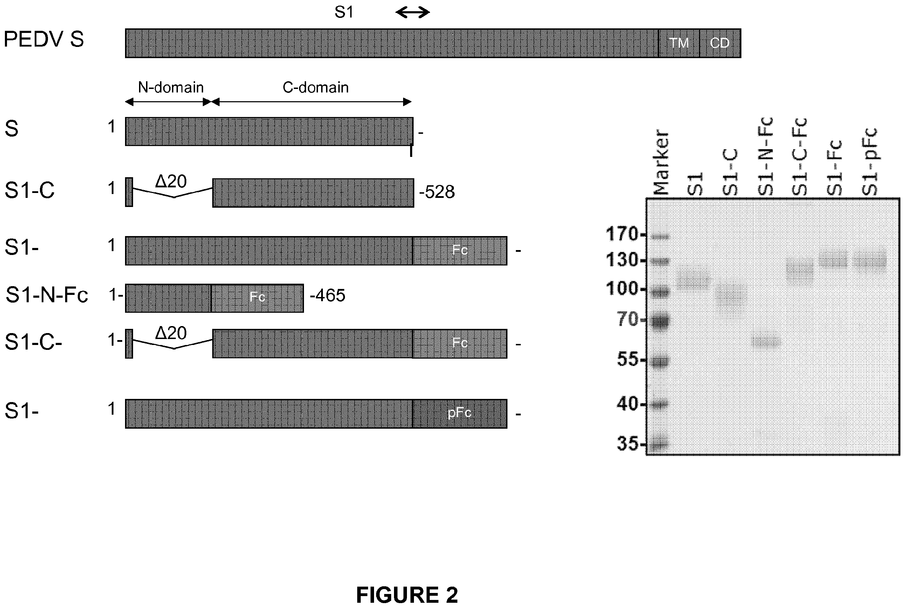

An overview of the S1 proteins is given in Figure 2. The subunit vaccines were formulated at 10 µg of S1 protein per 2 ml dose in XSolve.

### Killed virus vaccines

The KV vaccines contained killed whole PEDV, inactivated with 1 mM BEI. The strain, cell culture adpated D13, shares 93.3% of its amino acids with USA/Colorado/2013. The KV antigens were formulated as a ready-to-use formulations in XSolve.

### Virus neutralization assay

Sera were tested for the presence of neutralizing antibodies against PEDV using a virus neutralization (VN) assay on Vero cells. Briefly, serial two-fold dilutions of the test samples or reference sera were prepared in aMEM-TPB cell culture medium containing the antibiotics penicillin and streptomycin. Each dilution was mixed with an equal volume of 100 TCID₅₀ of an infectious PEDV strain that expresses green fluorescent protein (GFP) upon infection of susceptible Vero cells. After a pre-incubation period of 1 hour at 37°C, virus/serum dilution mixtures were transferred to 96-well microtitre plates containing Vero-CCL81 cells. After incubation for 48 hours at 37°C, the extent of GFP fluorescence indicative for virus replication was determined by microscopy. The antibody titre was calculated as log2 (per 50µl) of the reciprocal of the highest serum dilution where no virus replication could be demonstrated. Obtained VN titres are presented as averages calculated from duplicate measurements.

Pooled sera were tested in an alternative VN test that allows the use of either strain DR13 or strain GD01. Since GD01 requires trypsin for infection, the method described above was modified as follows: After co-incubation of 1 hour at 37°C, the virus/serum mixtures were left on the Vero-CCL81 cells for 2 hours at 37°C. Then the mixtures were removed, the cells were washed twice with PBS, and the cells were incubated at 37°C for 48 hours in the presence of aMEM-TPB plus 10 µg/ml trypsin for GD01 (or aMEM-TPB without trypsin in case strain DR13 was used in this procedure).

### PEDV antibody ELISA

Antibodies in sera against the S1 part of the PEDV spike protein derived from strain USA/Colorado/2013 were determined in an antibody ELISA. For this, plates were coated with 100 µl of PEDV S1 (0.25 µg/ml). Sera were diluted 1:900 in PBS0/1%BSA and added to the coated wells followed by incubation for 1 hour at 37°C. After two washes with PBS0/0.05%Tween-20, 100 µl of rabbit αSwine IgG HRPO conjugate diluted 1:10,000 in PBS0/1%BSA/0.5% tween20 was added to the wells, and plates were incubated for 1 hour at 37°C. After three washes with PBS0/0.05%Tween-20, 100µl/well of TMB "super slow" substrate was added to each well followed by incubation at room temperature for 10 min (in the dark). Reactions were stopped by adding 100 µl of 25% sulfuric acid per well. The optical density (OD) at 450nm was measured with an ELISA reader. Sera from naturally infected pigs were included as positive controls.

### RESULTS

### Body temperature

An overview of the mean body temperatures per group are shown in Table 4. It can be concluded that none of the vaccines caused a serious mean temperature elevation of more the 1 degrees Celsius. The largest temperature increase was seen at 4 hours post-vaccination, but in most cases the temperature returned to normal levels at 24 hours post-vaccination. The highest individual temperature increase (2.3°C) was observed for one animal in Group 6, 4h after the booster vaccination.

**Table 4. Mean rectal temperatures taken after the first or second vaccination.**

| | | **Temperature after 1st vaccination [°C]** | | | **Temperature after 2nd vaccination [°C]** | | |
|---|---|---|---|---|---|---|---|
| **Group** | **Vaccine** | **t=0h** | **t=4h** | **t=24h** | **t=0h** | **t=4h** | **t=24h** |
| 1a | US S1 | 39,60 | 39,68 | 39,84 | 39,74 | 40,52 | 39,64 |
| 1b | US S1-C | 39,34 | 39,92 | 39,52 | 39,50 | 40,02 | 39,78 |
| 2a | US S1-pFc | 39,60 | 40,18 | 39,90 | 39,98 | 40,18 | 39,70 |
| 2b | US S1-Fc | 39,86 | 40,08 | 39,90 | 39,82 | 40,26 | 39,48 |
| 3a | US S1-C-Fc | 39,60 | 39,96 | 40,14 | 39,46 | 40,22 | 39,44 |
| 3b | US S1-C-Fc | 39,58 | 39,62 | 39,80 | 39,72 | 40,10 | 39,56 |
| 4 | DR13 KV 6.0E5 | 39,28 | 40,04 | 39,55 | 39,84 | 40,49 | 39,57 |
| 5 | DR13 KV 3.0E6 | 39,51 | 39,92 | 39,62 | 39,60 | 40,05 | 39,46 |
| 6 | DR13 KV 1.5E7 | 39,25 | 39,94 | 39,77 | 39,84 | 39,81 | 39,61 |

### Local reactions

Animals were palpated on the site of intramuscular vaccine application at 2, 4, 6, 8, 10, 12, and 14 days post-vaccination. From the length and width (in cm), the surface of the local reaction (i.e. swelling) was calculated. One animal in Group 2b displayed a very mild (< 1 cm²) and transient (disappeared within a day) swelling directly after the booster vaccination. No local reactions were observed for all other 59 animals.

### Virus neutralizing antibodies

For the killed whole virus vaccines a virus neutralization assay was performed using the DR13 strain to be neutralized by the serum antibodies. The calculated mean VN titres of each group are presented in Table 5. From this table it can be concluded that DR13 KV antigen induced good VN titres in a clear dose responsive manner. It appears that a minimum dose corresponding to 3.0E6 TCID₅₀ killed whole PEDV is necessary to induce VN titres above 5 log 2. The result of this example confirms the results of Example 2.

**Table 5. PEDV neutralizing antibody titres (log₂/50µl).**

| **Group** | **Vaccine** | **t=4** | **t=5** | **t=6** |
|---|---|---|---|---|
| 4 | DR13 KV 6.0E5 | 2.8 | 3.9 | 3.3 |
| 5 | DR13 KV 3.0E6 | 5.5 | 6.0 | 5.5 |
| 6 | DR13 KV1.5E7 | 8.6 | 8.8 | 8.1 |

| | | | | |
|---|---|---|---|---|
| Time (t) is indicated in weeks after the first vaccination. | | | | |

For the subunit vacines the alternative VN test was used. This is because the spike protein of the DR13-GFP strain used in the other VN test is about 93.3% identical to that of the US isolates from which the S1 subunits were derived and approximately 92.7% identical to spike of strain GD01. This sequence difference might explain why VN antibodies cannot be detected in the other test. Therefore, sera were pooled per group and the presence of serum neutralizing antibodies was determined in the alternative VN test that allows the use of either the DR13-GFP or GD01 strain. The results are summarized in Table 6.

**Table 6. PEDV neutralizing antibody titres (log₂150µl) in pooled sera.**

| | | **PEDV strain DR13-GFP** | | | **PEDV strain GD01** | | |
|---|---|---|---|---|---|---|---|
| **Group** | **Vaccine** | **t=4** | **t=5** | **t=6** | **t=4** | **t=5** | **t=6** |
| 1a | US S1 | 0.0 | 0.0 | 0.0 | 6.0 | 5.5 | 4.5 |
| 1b | US S1-C | 0.0 | 0.0 | 0.0 | 5.0 | 5.5 | 4.0 |
| 2a | US S1-pFc | 0.0 | 0.0 | 2.0 | 7.0 | 8.0 | 8.0 |
| 2b | US S1-Fc | 1.0 | 0.0 | 0.0 | 6.0 | 8.0 | 8.0 |
| 3a | US S1-C-Fc | 1.0 | 0.0 | 0.0 | 8.0 | 6.0 | 7.5 |
| 3b | US S1-N-Fc | 0.0 | 0.0 | 0.0 | 1.0 | 0.0 | 1.0 |

| | | | | | | | |
|---|---|---|---|---|---|---|---|
| Time (t) is indicated in weeks after the first vaccination. | | | | | | | |

Indeed, the DR13 strain appears to be not suitable for detecting VN titres induced by the subunit vaccines. However, the DR13 neutralizing antibody titre in the pooled sera of the killed whole virus vaccines appeared to correspond well with the group averages of individually determined VN titres at 4, 5, and 6 weeks after the first vaccination (results no shown). This result demonstrates that (i) sera can be pooled to establish the VN titres per group, and (ii) the alternative VN assay for determining VN titres in pooled sera delivers similar results as the VN assay specifically developed for DR13-GFP. If the GD01 neutralizing antibody titre is determined, the picture for the S1 subunits (as shown in table 6, right hand side column) is as follows: With the exception of S1-N-Fc, they all induced a VN titre. The data show that (i) intact S1 or its C-terminal part are good antigens for triggering a VN antibody response, and (ii) a C-terminal extension with a Fc fragment improves the immune response.

Also, it is shown that 10µg of the S1 subunit is able to induce a VN titer that at least the same as the level that can be obtained with a vaccine comprising per dose 3E06 TCID₅₀ killed whole PED virus. Thus, 10µg of the S1 subunit is a dose that corresponds to at least 3E06 TCID₅₀ killed whole PED virus. To check this latter correspondence, a dose-response curve was made using the X-solve adjuvant and a dose range of 0.4µg - 2.0µg - 10µg - 50µg of the S1 subunit. After IM vaccination (prime-boost scheme with interval of three weeks) the lower dosages led to maximum VN titres (log₂/50µl, 6 weeks post vaccination) of approximately only 1, whereas the 10 µg group had a VN titer of 6.5 and the 50µg group had a VN titer of 7.0. This confirms that 10µg of the S1 subunit is a dose that corresponds to at least 3E06 TCID₅₀ killed whole PED virus (i.e. the established minimum dose able to induce a VN titre of at least 5).

### Antibodies against the S1 subunit of spike

An ELISA was performed on the pooled sera mentioned above for the last two time-points after vaccination. The ELISA data corresponded well with the VN titres (see Table 7).

**Table 7. OD₄₅₀ values of 1:900 diluted sera measured in the S1 ELISA.**

| **Group** | **Vaccine** | **t=5** | **t=6** |
|---|---|---|---|
| 1a | US S1 | 3.026 | 3.366 |
| 1b | US S1-C | 2.88 | 3.075 |
| 2a | US S1-pFc | 3.087 | 3.298 |
| 2b | US S1-Fc | 2.848 | 2.697 |
| 3a | US S1-C-Fc | 2.952 | 2.994 |
| 3b | US S1-N-Fc | 0.305 | 0.326 |

| | | | |
|---|---|---|---|
| Time (t) is indicated in weeks after the first vaccination. | | | |

## Claims

1. A vaccine for use in protecting offspring of a sow against an infection with porcine endemic diarrhea virus (PEDV), the vaccine comprising killed whole PEDV antigen and an oil containing adjuvant, wherein the oil is a non metabolizable oil and in that the oil is present in an amount less than 50% v/v, by administration of the vaccine to the sow at a dose of the antigen of at least 3.0E6 TCID₅₀ killed whole PEDV, defined as the dose of the antigen is such that at least a VN titre is obtained with the antigen that is the same as the VN titre that can be obtained with a vaccine comprising per dose 3.0E6 TCID₅₀ killed whole PED virus.

2. A vaccine for use according to claim 1, **characterised in that** the dose of the antigen is at least 1.5E7 TCID₅₀ killed whole PEDV.

3. A vaccine for use according to any of the preceding claims, **characterised in that** the total amount of the oil is less than 40% v/v.

4. A vaccine for use according to any of the preceding claims, **characterised in that** the oil is emulsified in an hydrophilic solvent.

5. A vaccine for use according to any of the preceding claims, **characterised in that** the vaccine in addition to the non-metabolizable oil comprises vitamin E acetate.

6. A vaccine for use to protect offspring of a sow against an infection with porcine endemic diarrhea virus (PEDV) by administering a vaccine to the sow, the vaccine comprising killed whole PEDV antigen and an oil containing adjuvant, wherein the oil is a non metabolizable oil and wherein the total amount of oil in the vaccine is less than 50% v/v, and the antigen is present in a concentration of at least 1.5E6 TCID50 killed whole PEDV per ml, defined as the dose of the antigen is such that at least a VN titre is obtained with the antigen that is the same as the VN titre that can be obtained with a vaccine comprising per dose 1.5E6 TCID₅₀ killed whole PED virus.

## Patentansprüche

1. Impfstoff zur Verwendung beim Schützen von Nachkommen einer Sau gegen eine Infektion mit PEDV (Porcine Endemic Diarrhea Virus), wobei der Impfstoff abgetötetes ganzes PEDV als Antigen und ein ölhaltiges Adjuvans umfasst, wobei es sich bei dem Öl um ein nicht metabolisierbares Öl handelt und in dem das Öl in einer Menge von weniger als 50 Vol.-% vorliegt, durch Verabreichung des Impfstoffs an die Sau in einer Dosis des Antigens von wenigstens 3,0E6 TCID₅₀ abgetötetes ganzes PEDV, definiert als die Dosis des Antigens, die so beschaffen ist, dass wenigstens ein VN-Titer mit dem Antigen erhalten wird, der mit dem VN-Titer identisch ist, der sich mit einem Impfstoff erhalten lässt, der pro Dosis 3,0E6 TCID₅₀ abgetötetes ganzes PED-Virus umfasst.

2. Impfstoff zur Verwendung nach Anspruch 1, **dadurch gekennzeichnet, dass** die Dosis des Antigens wenigstens 1,5E7 TCID₅₀ abgetötetes ganzes PEDV beträgt.

3. Impfstoff zur Verwendung nach einem der vorhergehenden Ansprüche, **dadurch gekennzeichnet, dass** die Gesamtmenge des Öls weniger als 40 Vol.-% beträgt.

4. Impfstoff zur Verwendung nach einem der vorhergehenden Ansprüche, **dadurch gekennzeichnet, dass** das Öl in einem hydrophilen Lösungsmittel emulgiert ist.

5. Impfstoff zur Verwendung nach einem der vorhergehenden Ansprüche, **dadurch gekennzeichnet, dass** der Impfstoff neben dem nicht metabolisierbaren Öl Vitamin-E-Acetat umfasst.

6. Impfstoff zur Verwendung zum Schutz von Nachkommen einer Sau gegen eine Infektion mit PEDV (Porcine Endemic Diarrhea Virus) durch Verabreichen eines Impfstoffs an die Sau, wobei der Impfstoff abgetötetes ganzes PEDV als Antigen und ein ölhaltiges Adjuvans umfasst, wobei es sich bei dem Öl um ein nicht metabolisierbares Öl handelt und wobei die Gesamtmenge des Öls im Impfstoff weniger als 50 Vol.-% beträgt und das Antigen in einer Konzentration von wenigstens 1,5E6 TCID₅₀ abgetötetes ganzes PEDV pro ml vorliegt, definiert als die Dosis des Antigens, die so beschaffen ist, dass wenigstens ein VN-Titer mit dem Antigen erhalten wird, der mit dem VN-Titer identisch ist, der sich mit einem Impfstoff erhalten lässt, der pro Dosis 1,5E6 TCID₅₀ abgetötetes ganzes PED-Virus umfasst.

## Revendications

1. Vaccin pour une utilisation dans la protection d'une progéniture d'une truie contre une infection par le virus de la diarrhée endémique porcine (PEDV), le vaccin comprenant un antigène de PEDV entier tué et un adjuvant contenant une huile, l'huile étant une huile non métabolisable et en ce que l'huile est présente en une quantité inférieure à 50 % v/v, par administration du vaccin à la truie à une dose de l'antigène d'au moins 3,0 . 10⁶ TCID₅₀ de PEDV entier tué, définie comme la dose de l'antigène étant telle qu'au moins un titre VN est obtenu avec l'antigène qui est le même que le titre VN qui peut être obtenu avec un vaccin comprenant par dose 3,0 . 10⁶ TCID₅₀ de virus PED entier tué.

2. Vaccin pour une utilisation selon la revendication 1, **caractérisé en ce que** la dose de l'antigène est d'au moins 1,5 . 10⁷ TCID₅₀ de PEDV entier tué.

3. Vaccin pour une utilisation selon l'une quelconque des revendications précédentes, **caractérisé en ce que** la quantité totale de l'huile est inférieure à 40 % v/v.

4. Vaccin pour une utilisation selon l'une quelconque des revendications précédentes, **caractérisé en ce que** l'huile est émulsifiée dans un solvant hydrophile.

5. Vaccin pour une utilisation selon l'une quelconque des revendications précédentes, **caractérisé en ce que** le vaccin en plus de l'huile non métabolisable comprend de l'acétate de vitamine E.

6. Vaccin pour une utilisation pour protéger une progéniture d'une truie contre une infection par le virus de la diarrhée endémique porcine (PEDV) par administration d'un vaccin à la truie, le vaccin comprenant un antigène de PEDV entier tué et un adjuvant contenant une huile, l'huile étant une huile non métabolisable et la quantité totale d'huile dans le vaccin étant inférieure à 50 % v/v, et l'antigène étant présent en une concentration d'au moins 1,5 . 10⁶ TCID50 de PEDV entier tué par ml, définie comme la dose de l'antigène étant telle qu'au moins un titre VN est obtenu avec l'antigène qui est le même que le titre VN qui peut être obtenu avec un vaccin comprenant par dose 1,5 . 10⁶ TCID₅₀ de virus PED entier tué.
